(19) Europäisches Patentamt
European Patent Office
Office européen des brevets

(11) Publication number: **0 521 465 A2**

(12) # EUROPEAN PATENT APPLICATION

(21) Application number: **92111068.0**

(22) Date of filing: **30.06.92**

(51) Int. Cl.[5]: **C07D 265/30**, A01N 43/84, C07C 217/22, C07C 225/16, A01N 35/04, A01N 33/08

(30) Priority: **01.07.91 IT MI911812**

(43) Date of publication of application: **07.01.93 Bulletin 93/01**

(84) Designated Contracting States: **AT BE CH DE DK ES FR GB GR IT LI LU MC NL PT SE**

(71) Applicant: **MINISTERO DELL' UNIVERSITA' E DELLA RICERCA SCIENTIFICA E TECNOLOGICA**
**76, Lungotevere Thaon di Revel**
**I-00196 Roma(IT)**

(72) Inventor: **Camaggi, Giovanni**
**Via Ragazzi del 99, 20**
**I-28100 Novara(IT)**
Inventor: **Filippini, Lucio**
**Via R. Morandi 13/A**
**I-20097 San Donato Milanese (Milan)(IT)**
Inventor: **Gusmeroli, Marilena**
**Via della Guerrina, 31**
**I-20052 Monza (Milano)(IT)**
Inventor: **Riva, Raul**
**Baluardo d'Azeglio, 5**
**I-28100 Novara(IT)**
Inventor: **Garavaglia, Carlo**
**Piazza della Vittoria, 7**
**I-20012 Cuggiono (Milano)(IT)**
Inventor: **Mirenna, Luigi**
**Via Gamboloita, 4**
**I-20100 Milano(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

(54) **Anti-fungal benzophenones.**

(57) Described are benzophenones showing an antifungal activity and corresponding to general formula (I):

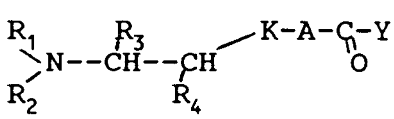

(I)

wherein $R_1$ to $R_4$, K, A and Y have the meanings given in claim 1.

EP 0 521 465 A2

The present invention relates to benzophenones showing high antifungal activity, to a process for their preparation and to their use as fungicides in the field of agriculture.

In particular the present invention relates to compounds of general formula (I):

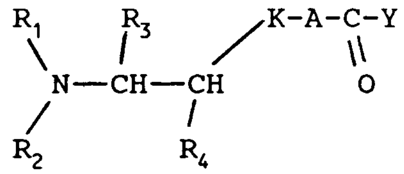

(I)

wherein:

$R_1$ and $R_2$, the same or different, represent $C_1$-$C_6$ (preferably $C_1$-$C_3$) alkyl groups or $C_7$-$C_{10}$ aralkyl groups (e.g. methyl, ethyl, benzyl, phenethyl and 3-phenyl propyl); or $R_1$ and $R_2$, together with the N atom to which they are bound, represent a heterocyclic $C_3$-$C_8$ group or a heterocyclic $C_2$-$C_7$ group containing a second hetero-atom selected from O and S, said heterocyclic groups being optionally substituted with one or more (e.g. two) $C_1$-$C_4$ alkyl groups (e.g. methyl or ethyl);

$R_3$ and $R_4$, the same or different, represent H and $C_1$-$C_3$ alkyl groups (preferably H or methyl);

K represents an oxygen atom or a methylene group;

A represents a phenylene group (preferably a 1,3-phenylene group), optionally substituted with one or more groups selected from halogen atoms, $C_1$-$C_4$ alkyl and haloalkyl groups and $C_1$-$C_4$ alkoxy and haloalkoxy groups;

Y represents a $C_6$-$C_{10}$ aryl group (preferably phenyl); said group may optionally be substituted with one or more groups selected from halogen atoms, $C_1$-$C_4$ alkyl and haloalkyl groups and $C_1$-$C_4$ alkoxy and haloalkoxy groups.

The compounds corresponding to formula (I) have at least one asymmetrical centre: the present invention includes both enantiomerically or diastereomerically pure compounds as well as mixtures thereof in any ratio.

In the above definition, halogen atoms are F,Cl, Br or I atoms.

Examples of aryl groups are phenyl, naphthyl and similar higher groups.

Preferred examples of aralkyl groups are benzyl and 3-phenylpropyl.

Preferred examples of groups $R_1R_2N$-, when taken together, are groups derived from morpholine, piperidine, thiomorpholine, etc., also substituted as defined above.

The present invention also relates to:

- the salts of compounds of general formula (I) derived from inorganic acids such as a hydrogen halide (for example, hydroiodic and hydrobromic acid); sulphuric acid, nitric acid, thiocyanic acid and phosphoric acid; and from organic acids such as acetic acid, propanoic acid, ethane dioic acid, propane dioic acid, benzoic acid, salicylic acid, saccharin, methanesulphonic acid, 4-methylbenzenesulphonic acid, etc.;
- metal complex compounds obtained by complex-forming reactions between the derivatives (I) and an organic or inorganic metal salt such as a halide, nitrate, sulphate, phosphate of, for example, copper, manganese, zinc and iron.

The compounds corresponding to formula (I) can be prepared by means of various synthesis schemes.

A preferred method may schematically be represented as follows:

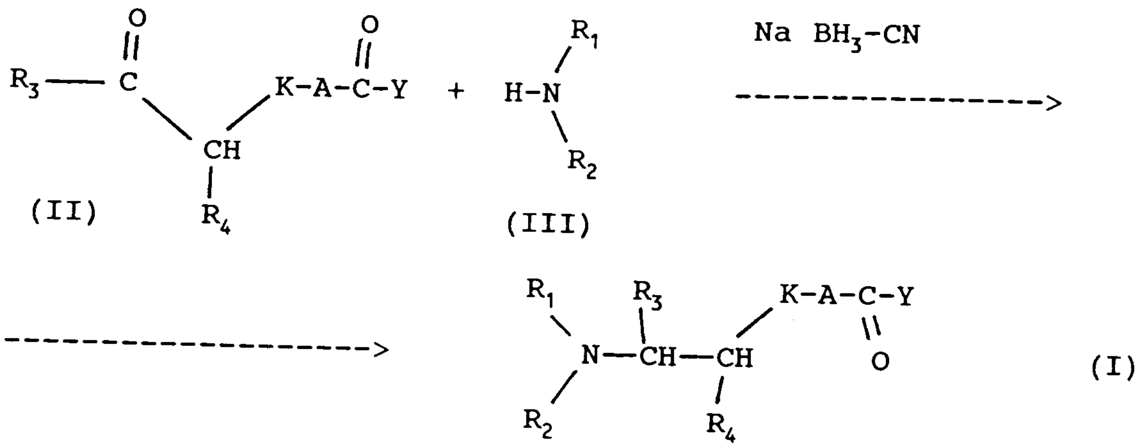


(II) (III) (I)

More specifically, the carbonyl compound (II) is reacted with the amine (III) and cyano sodium borohydride, preferably in a protic solvent (e.g. methanol, ethanol) and at a temperature of from -5 to 25 ° C, to obtain compound (I) (cf. Organic Synthesis, Vol. 52, page 124).

In the above scheme, the symbols $R_1$, $R_2$, $R_3$, $R_4$, K, A and Y have the meanings already defined.

The salts and/or complex compounds can be prepared from compounds (I) by following the known methods.

The amines (III) are readily available commercially or can easily be obtained by synthesis (cf. J. March, "Advanced Organic Chemistry", II edition, Int. St. Edition, page 357).

The carbonyl compounds (II) can generally be prepared by using the known techniques.

When K is a methylene group, a preferred method may schematically be represented as follows:

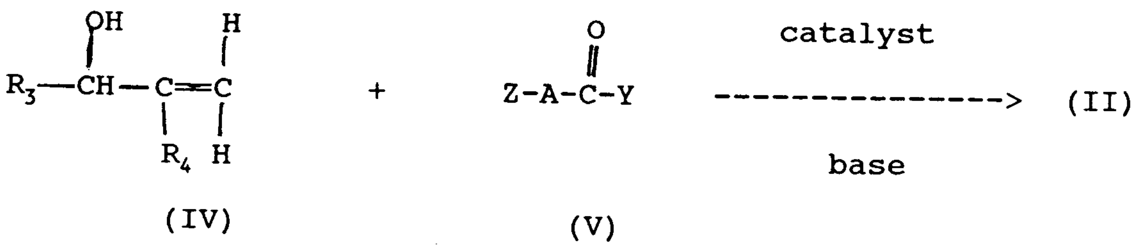


(IV) (V) (II)

More specifically, the allyl alcohol (IV) is reacted with the benzophenone (V), wherein Z represents halogen (e.g. Br, I) or an activated ester (e.g. trifluoromethansulphonic), in the presence of a palladium salt (e.g. Pd chloride, Pd acetate), or in the presence of palladium metal supported, for example, on carbon or inorganic salts (e.g. sodium bicarbonate, sodium carbonate). These forms of metallic palladium are often more effective if prepared "in situ". The reaction is frequently carried out in the presence of an organic base (e.g. triethylamine, tributylamine) or an inorganic base (e.g. sodium bicarbonate, potassium carbonate) and in a protic solvent (e.g. water, ethanol) or bipolar aprotic solvent (e.g. N,N-dimethylformamide, N-methylpyrrolidone), at a temperature ranging from 0 ° C to the boiling point of the solvent. It might be advantageous to add phosphines such as triphenylphosphine and triorthotolylphosphine (cf. JOC 41, 1206, 1976).

In the preparation scheme (II), $R_3$, $R_4$, A and Y have the meanings already defined.

The alcohols (IV) and the benzophenones (V) can generally be prepared by using the known methods.

The compounds corresponding to general formula (I) are highly active in inhibiting the growth of various species of pathogenous fungi which attack cultivations of useful plants.

They have both a preventive and curative activity when applied to useful plants or parts of these, such as leaves, and are particularly effective in preventing diseases caused by pathogenous fungi, such as, for example, those belonging to the Helminthosporium genera.

Examples of plant diseases which can be combatted with the compounds of the present invention are:

- Erysiphe graminis on cereals
- Sphaeroteca fuliginea on cucurbitaceae (for example, cucumber)
- Puccinia on cereals
- Septoria on cereals
- Helminthosporium on cereals

- Rhynchosporium on cereals
- Podosphaera leucotricha on apple-trees
- Uncinula necator on vines
- Venturia inaequalis on apple-trees
- Pyricularia oryzae on rice
- Botrytis cinerea
- Fusarium on cereals

and other diseases.

For practical use in agriculture, it is often useful to prepare fungicidal compositions containing one or more of the compounds corresponding to formula (I) as active substance, optionally along with other active ingredients.

These compositions may be applied to any part of the plant, for example, leaves, stems, branches and roots, or on the seeds themselves, before sowing, or even to the soil in which the plants grow. Compositions in the form of dry powders, wettable powders, emulsifiable concentrates, pastes, granulates, solutions, suspensions etc. may be used; the choice of the type of composition will depend on the intended use. The compositions may be prepared according to the known techniques, such as diluting or dissolving the active substance with a solvent medium and/or solid diluent, optionally in the presence of surface-active agents. The following are examples of solid diluents or supports which may be used: silica, kaolin, bentonite, talc, infusorial earth, dolomite, calcium carbonate, magnesia, chalk, clays, synthetic silicates, attapulgite, sepiolite. As liquid diluents, apart from water, various kinds of solvents may be used, for example aromatics (e.g. benzene, xylenes, or mixtures of alkylbenzenes), chloroaromatics (e.g. chlorobenzene), paraffins (e.g. petroleum fractions), alcohols (e.g. methanol, propanol, butanol), amines, amides (e.g. dimethylformamide), ketones (e.g. cyclohexanone, acetophenone, isophorone, ethyl amyl ketone), esters (e.g. isobutyl acetate). Examples of suitable surface-active agents are sodium, calcium and triethanolamine salts of alkylsulphates, alkylsulphonates, alkyl-arylsulphonates, polyethoxylated alkylphenols, fatty alcohols condensed with ethylene oxide, polyoxyethylated fatty acids, esters of polyoxyethylated sorbitol, polyoxyethylated fats and ligninsulphonates. The compositions may also contain special additives for specific purposes, for example, adhesives such as gum arabic, polyvinyl alcohol and polyvinyl pyrrolidone.

If required, it is also possible to add to the compositions of the present invention other compatible active substances such as fungicides, agrochemicals, phyto-regulators, weed-killers, insecticides and fertilizers.

The concentration of the active substance in the above compositions may vary within a wide range, depending on the active compound, the cultivation, the pathogen, the environmental conditions and the type of formulation used. In general the concentration of the active substance ranges from 0.1 to 95, preferably from 0.5 to 90% by weight, based on the composition.

The following examples are to further illustrate the present invention without limiting the scope thereof.

EXAMPLE 1

Synthesis of 3-(3-benzoylphenyl)-2-methylpropanal.

0.085 g of palladium chloride, 0.254 g of triphenylphosphine and 16.9 g of sodium bicarbonate are dispersed in 35 ml of N-methylpyrrolidone. Hydrogen is introduced into the reaction flask for 30 min., under vigorous stirring. The hydrogen is removed by repeated purging operations with nitrogen and 10 g of 3-bromobenzophenone and 3.75 g of 2-methyl-prop-2-en-l-ol are then added.

The mixture is heated to 120 °C for 0.5 hours.

At the end of the reaction, the solution is filtered on celite, 80 ml of water are added and extraction takes place with diethyl ether (2x50 ml). The organic phase, after evaporation of the solvent under reduced pressure, is dried. The crude product is purified by silica gel chromatography, with hexane/ethyl acetate = 9/1 as eluent, to obtain 8.9 g of the title product.

| $^1$H-NMR (60 MHz) in $CDCl_3$: | |
|---|---|
| 9.8 | (s, 1H) |
| 8.0 - 7.0 | (m, 9H) |
| 3.2 - 2.6 | (m, 3H) |
| 1.2 | (d, 3H) |
| 1.3 - 0.8 | (m, 9H). |

EXAMPLE 2

Synthesis of 4-[3-(3-benzoylphenyl)-2-methylpropyl]-2,6-dimethylmorpholine (Compound No. 1).

0.7 g of 2,6-dimethylmorpholine hydrochloride, a mixture of cis-trans isomers, are dissolved in 8 ml of methanol, whereafter 0.069 g of potassium hydroxide and 1 g of 3-(3-benzoylphenyl)-2-methylpropanal are added. After 15 minutes a solution of 0.084 g of sodium cyano borohydride in 1 ml of methanol is added dropwise and after a further 30 minutes 0.27 g of potassium hydroxide in powder form are added. The mixture is filtered on celite and the solvent evaporated at reduced pressure. The crude product is purified by silica gel chromatography, with hexane/ethyl acetate = 9/1 as eluent, to obtain 0.8 g of compound 1, cis isomer.

| $^1$H-NMR (60 MHz) in $CDCl_3$: | |
|---|---|
| 8.0 - 7.2 | (m, 9H) |
| 3.5 | (m, 2H) |
| 3.0 - 1.4 | (m, 9H) |
| 1.3 - 0.8 | (m, 9H). |

EXAMPLE 3

Following the same procedure as described in example 2, starting from the corresponding reagents, the compounds No. 2-9 were synthesized and the NMR analytic characteristics are given below:

Compound 2

4-{3-[3-(4-Chlorobenzoyl)phenyl]-2-methylpropyl}-2,6-dimethylmorpholine (cis isomer).

| $^1$H-NMR (60 MHz) in $CDCl_3$: | |
|---|---|
| 7.9 - 7.2 | (m, 9H) |
| 3.5 | (m, 2H) |
| 3.0 - 1.4 | (m, 9H) |
| 1.3 - 0.8 | (m, 9H). |

Compound 3

4-{3-[3-(4-Fluorobenzoyl)phenyl]-2-methylpropyl}-2,6-dimethylmorpholine (cis isomer).

| $^1$H-NMR (60 MHz) in $CDCl_3$: | |
|---|---|
| 8.0 - 6.9 | (m, 9H) |
| 3.5 | (m, 2H) |
| 2.9 - 1.4 | (m, 9H) |
| 1.2 - 0.7 | (m, 9H). |

Compound 4

4-[3-(3-Benzoylphenyl)-2-methyl-3-oxapropyl]-2,6-dimethylmorpholine(cis isomer).

| $^1H$-NMR (60 MHz) in $CDCl_3$: | |
|---|---|
| 8.0 - 7.0 | (m, 9H) |
| 4.6 | (m, 1H) |
| 3.6 | (m, 2H) |
| 2.5 | (m, 4H) |
| 1.8 | (m, 2H) |
| 1.3 | (m, 3H) |
| 1.1 | (m, 6H) |

Compound 5

N-[3-(3-Benzoylphenyl)-2-methyl-3-oxapropyl]-N-benzyl-N-methylamine.

| $^1H$-NMR (60 MHz) in $CDCl_3$: | |
|---|---|
| 8.0 - 7.0 | (m, 9H) |
| 4.6 | (m, 1H) |
| 3.6 | (s, 2H) |
| 2.7 | (m, 2H) |
| 2.3 | (s, 3H) |
| 1.35 | (d, 3H) |

Compound 6

4-{3-[3-(3,4-Dimethoxybenzoyl)phenyl]-2-methylpropyl}-2,6-dimethylmorpholine (cis isomer).

| $^1H$-NMR (60 MHz) in $CDCl_3$: | |
|---|---|
| 7.5 - 6.7 | (m, 7H) |
| 3.8 | (s, 6H) |
| 3.5 | (m, 2H) |
| 1.4 - 2.8 | (m, 9H) |
| 0.6 - 1.2 | (m, 9H) |

Compound 7

N-[3-(3-Benzoylphenyl)-2-methylpropyl]-N-methyl-N-(3-phenylpropyl)amine.

| $^1H$-NMR (60 MHz) in $CDCl_3$: | |
|---|---|
| 7.8 - 7.1 | (m, 14H) |
| 1.5 - 2.75 | (m, 11H) |
| 2.1 | (s, 3H) |
| 0.9 | (d, 3H) |

Compound 8

N-[3-(3-Benzoylphenyl)-2-methylpropyl]-N-methyl-N-benzyl amine.

| $^1$H-NMR (60 MHz) in $CDCl_3$: | |
|---|---|
| 7.9 - 7.1 | (m, 14H) |
| 3.5 | (s, 2H) |
| 3.1 - 1.6 | (m, 5H) |
| 2.1 | (s, 3H) |
| 0.9 | (d, 3H) |

Compound 9

N-[3-(3-Benzoylphenyl)-2-methylpropyl]-N,N-dipropyl amine.

| $^1$H-NMR (60 MHz) in $CDCl_3$: | |
|---|---|
| 7.8 - 6.8 | (m, 9H) |
| 3.1 - 1.0 | (m, 13H) |
| 0.8 | (m, 9H) |

EXAMPLE 4

Determination of the preventive antifungal activity on Helminthosporium teres.

Barley leaves cv. Arna, cultivated in a vase in a conditioned environment, were treated by spraying both sides thereof with the products being tested (compounds No. 1 and 2) in a 20% aqueous solution of acetone (vol./vol.).

After having stayed for 2 days in a conditioned environment at 20 °C and 70% R.H., the plants were sprayed on both sides of the leaves with an aqueous suspension of Helminthosporium teres (250,000 conidii per ml). After 24 hours in a humidity saturated atmosphere, at 21 °C, the plants were kept in a conditiond environment for the incubation of the fungus.

At the end of this period (12 days), the severity of infection was assessed by observation, with a rating scale ranging from 100 (healthy plant) to 0 (completely infected plant).

The obtained results are summarized in Table 1.

TABLE 1

| Compound No. | Dosage (ppm) | % Control Helminthosporium |
|---|---|---|
| 1 | 500<br>125 | 100<br>100 |
| 2 | 500<br>125 | 100<br>100 |

**Claims**

**1.** Anti-fungal benzophenones of general formula (I):

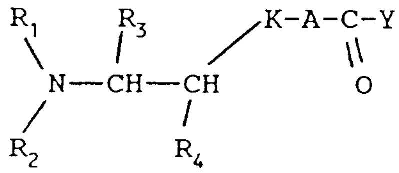

(I)

wherein:
$R_1$ and $R_2$, the same or different, represent $C_1$-$C_6$ alkyl or $C_7$-$C_{10}$ aralkyl groups;
or $R_1$ and $R_2$, together with the N to which they are linked, represent a heterocyclic $C_3$-$C_8$ group or a heterocyclic $C_2$-$C_7$ group containing a second hetero-atom selected from O and S, said heterocyclic groups being optionally substituted with one or more $C_1$-$C_4$ alkyl groups;
$R_3$ and $R_4$, the same or different, represent H or $C_1$-$C_3$ alkyl groups;
K represents oxygen or a methylene group;
A represents a phenylene group, optionally substituted with one or more groups selected from halogen, $C_1$-$C_4$ alkyl, $C_1$-$C_4$ haloalkyl, $C_1$-$C_4$ alkoxy and $C_1$-$C_4$ haloalkoxy groups;
Y represents a $C_6$-$C_{10}$ aryl group, said group being optionally substituted with one or more groups selected from halogen, $C_1$-$C_4$ alkyl, $C_1$-$C_4$ haloalkyl, $C_1$-$C_4$ alkoxy and $C_1$-$C_4$ haloalkoxy groups;
and salts and metal complex compounds thereof.

**2.** Compounds according to claim 1, wherein said heterocyclic $C_3$-$C_8$- or $C_2$-$C_7$ groups are selected from morpholine, piperidine and thiomorpholine groups, said groups being optionally substituted, preferably by at least one $C_1$-$C_4$ alkyl group.

**3.** Compounds according to any one of claims 1 and 2, wherein $R_3$ is H and $R_4$ is methyl.

**4.** Compounds according to any one of claims 1 to 3, wherein A represents an unsubstituted phenylene group.

**5.** Compounds according to any one of claims 1 to 4, wherein Y represents an optionally substituted phenyl group.

**6.** Compounds according to claim 1, namely 4-[3-(3-benzoylphenyl)-2-methylpropyl]-2,6-dimethylmorpholine (cis isomer); 4-{3-[3-(4-chlorobenzoyl)phenyl]-2-methylpropyl}-2,6-dimethylmorpholine (cis isomer); 4-{3-[3-(4-fluorobenzoyl)phenyl]-2-methylpropyl}-2,6-dimethylmorpholine (cis isomer); 4-[3-(3-benzoylphenyl)-2-methyl-3-oxapropyl]-2,6-dimethylmorpholine (cis isomer); N-[3-(3-benzoylphenyl)-2-methyl-3-oxapropyl]-N-benzyl-N-methylamine; 4-{3-[3-(3,4-dimethoxybenzoyl)phenyl]-2-methylpropyl}-2,6-dimethylmorpholine (cis isomer); N-[3-(3-benzoylphenyl)-2-methylpropyl]-N-methyl-N-(3-phenylpropyl)amine; N-[3-(3-benzoylphenyl)-2-methylpropyl]-N-methyl-N-benzylamine; and N-[3-(3-benzoylphenyl)-2-methylpropyl]-N,N-dipropylamine.

**7.** Process for the preparation of the compounds according to any one of the preceding claims, comprising the reaction of a carbonyl compound of general formula (II):

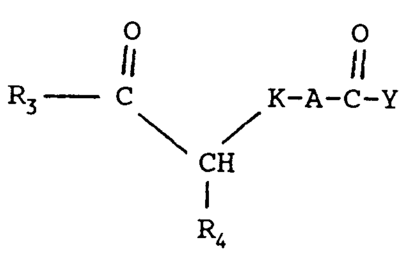

(II)

with sodium cyanoborohydride and an amine of general formula (III):

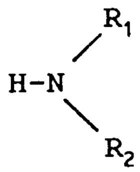

(III)

wherein $R_1$ to $R_4$ K, A and Y are as defined in claim 1.

**8.** Process according to claim 7, wherein the reaction is carried out at a temperature of from -5 to +25 °C and/or in a protic solvent, preferably selected from methanol and ethanol.

**9.** Compositions suitable for inhibiting the growth of pathogenous fungi in the cultivation of useful plants, comprising one or more compounds of general formula (I) as defined in claim 1, preferably in the form of dry powders, wettable powders, emulsifiable concentrates, pastes, granulates, solutions or suspensions.

**10.** Compositions according to claim 9, suitable for inhibiting the growth of fungi belonging to the Helminthosporium genera.